# EUROPEAN PATENT SPECIFICATION

(11) **EP 1 857 052 B1**
(45) Date of publication and mention of the grant of the patent: **02.09.2009**
(21) Application number: 07017704.3
(22) Date of filing: 17.02.2005
(51) Int. Cl.: A61B 17/00, A61B 17/12

(54) **Devices for closing a patent foramen ovale**
Vorrichtungen zur Schließung eines Patent-Foramenovals
Dispositifs pour la fermeture de foramen ovale persistant

(30) Priority: 20.02.2004 US 783783; 07.05.2004 US 841880
(43) Date of publication of application: 21.11.2007
(62) Divisional of application: 05713631.9
(73) Proprietor: ev3 Endovascular, Inc., Plymouth, MN 55442 (US)
(72) Inventor: Klenk, Alan R., San Jose CA 95129 (US); Le, Hai Q., San Jose CA 95121 (US); Han, Thuzar K., Fremont CA 94538 (US); Fraizer, Andrew G. C., Sunnyvale, CA 94087 (US); Roue Chad C., San Jose, CA 95129 (US)
(74) Representative: Mallalieu, Catherine Louise

(56) References cited:
- WO-A-99/12480
- US-A1- 2002 151 921
- US-B1- 6 482 224

## Description

### Background of the Invention

### Field of the Invention

The present invention relates to devices for closing a body lumen or cavity and, in particular, for closing a patent foramen ovale.

### Description of the Related Art

Embolic stroke is the nation's third leading killer for adults, and is a major cause of disability. There are over 700,000 strokes per year in the United States alone. Of these, roughly 100,000 are hemorrhagic, and 600,000 are ischemic (either due to vessel narrowing or to embolism). About 50,000 of the ischemic strokes are believed to be caused by a patent foramen ovale. However, the risk of recurrent stroke is higher in patients whose strokes are caused by a patent foramen ovale.

Pharmacological therapies for stroke prevention such as oral or systemic administration of warfarin or the like have been inadequate due to serious side effects of the medications and lack of patient compliance in taking the medication.

In general, the heart is divided into four cambers, the two upper being the left and right atria and the two lower being the left and right ventricles. The atria are separated from each other by a muscular wall, the interatrial septum, and the ventricles by the interventricular septum.

Either congenitally or by acquisition, abnormal openings, holes or shunts can occur between the chambers of the heart or the great vessels (interatrial and interventricular septal defects or patent ductus arteriosus and aortico-pulmonary window respectively), causing shunting of blood through the opening. During fetal life, most of the circulating blood is shunted away from the lungs to the peripheral tissues through specialized vessels and foramens that are open ("patent"). In most people these specialized structures quickly close after birth, but sometimes they fail to close. A patent foramen ovale is a condition wherein an abnormal opening is present in the septal wall between the two atria of the heart. An atrial septal defect is a condition wherein a hole is present in the septal wall between the two atria of the heart.

In contrast to other septal defects which tend to have a generally longitudinal axis, a patent foramen ovale tends to behave like a flap valve. Accordingly, the axis of the patent foramen ovale tends to be at an angle, and almost parallel to the septal wall. The patent foramen ovale is a virtual tunnel, long and wide, but not very tall. It is normally closed because the roof and floor of the tunnel are in contact, but it can open when the pressure in the right side of the heart becomes elevated relative to the pressure in the left side of the heart, such as while coughing

Studies have shown that adults with strokes of unknown origin (cryptogenic strokes) have about twice the normal rate of patent foramen ovales than the normal population. Although there is a correlation between strokes and patent foramen ovales, it is currently unknown why this correlation exists. Many people theorize that blood clots and plaque that have formed in the peripheral venous circulation (in the legs for example) break off and travel to the heart. Normally, the clots and plaque get delivered to the lungs where it is trapped and usually cause no harm to the patient. Patients with a patent foramen ovale, however, have a potential opening that the clots or plaque can pass through the venous circulation and into the arterial circulation and then into the brain or other tissues to cause a thromboembolic event like a stroke. The clots may pass to the arterial side when there is an increase in the pressure in the right atrium. Then the clots travel through the left side of the heart, to the aorta, and then to the brain via the carotid arteries where they cause a stroke and the associated neurological deficits.

Previously, patent foramen ovale have required relatively extensive surgical techniques for correction. To date the most common method of closing intracardiac shunts, such as a patent foramen ovale, entails the relatively drastic technique of open-heart surgery, requiring opening the chest or sternum and diverting the blood from the heart with the use of a cardiopulmonary bypass. The heart is then opened, the defect is sewn shut by direct suturing with or without a patch of synthetic material (usually of Dacron, Teflon, silk, nylon or pericardium), and then the heart is closed. The patient is then taken off the cardiopulmonary bypass machine, and then the chest is closed.

In place of direct suturing, closure of a patent foramen ovale by means of a mechanical prosthesis has also been disclosed. A number of devices designed for closure of interauricular septal defects have been used to correct patent foramen ovale.

Although these devices have been known to effectively close other septal defects, there are few occlusion devices which have been developed specifically for closing patent foramen ovale. Although these devices have been effective in some cases, there is still much room for improvement.

Notwithstanding the foregoing, there remains a need for a transluminal method and improved apparatus for correcting patent foramen ovale.

US 6, 482 224 discloses a flexible endovascular stapling device for an intravascular procedure such as patent foramen ovale closure according to the preamble of claim 1.

### Summary of the Invention

The present invention provides improved delivery and positioning systems for delivering a closure device to a patent foramen ovale.

According to the present invention there is provided an assembly for delivering a closure device to a patent foramen ovale having a septum primum and a septum secundum, comprising:
a closure device suitable for deployment at the patent foramen ovale to hold the septum primum and septum secundum together;
a delivery catheter sized to receive the closure device and suitable for advancing the closure device to the patent foramen ovale from one side of the patent foramen ovale; and **characterized in that** the assembly further comprises
a countertraction element suitable for positioning on the opposite side of the patent foramen ovale, the countertraction element suitable for holding the position of at least one of the septa while the closure device is advanced into position.

We describe a method of closing a patent foramen ovale having a septum primum and a septum secundum. An elongate body having a proximal end and a distal end is delivered to the patent foramen ovale. The elongate body has a tissue piercing structure at its distal end and a coil releasably engaged with the elongate body. The tissue piercing structure and the coil are advanced through the septa of the patent foramen ovale. The coil is released from the elongate body and the tissue piercing structure is withdrawn from the septa of the patent foramen ovale, the coil when released contracting to pinch the septum primum and the septum secundum together.

The elongate body may include an opening near its distal end, and the coil may have a distal end that releasably engages the opening in the elongate body near its distal end. A loading portion may be provided to releasably engage a proximal end of the coil, the coil being advanced through the septa of the patent foramen ovale while the coil is engaged with both the loading portion and the opening near the distal end of the elongate body to axially elongate and radially reduce the coil. In one embodiment, a loading collar is delivered with the elongate body to the patent foramen ovale, the loading collar releasably engaging a proximal end of the coil. The elongate body may be rotatable relative to the loading collar, and/or may be axially slideably relative to the loading collar.

We also describe a method of closing a patent foramen ovale having a septum primum and septum secundum comprises advancing a plurality of coils at least partially through the septa of the patent foramen ovale to secure the septum primum and septum secundum together. The plurality of coils may be advanced sequentially through a single catheter. Each of the coils may be provided over a single elongate body and be advanced through the septa of the patent foramen ovale using a tissue piercing structure on the distal end of the elongate body. In one embodiment, at least three coils are advanced through the septa of the patent forman ovale.

We also describe as assembly for delivering a coil through tissue in a patient is provided. The assembly comprises a loading portion adapted to releasably engage a proximal end of the coil. A tissue piercing structure is also provided adapted to releasably engage a distal end of the coil The loading portion holds the coil relative to the tissue piercing structure to axially elongate and radially reduce the coil. In one embodiment, the loading portion is integral with the tissue piecing structure. The loading portion may have a rectangular shape. The loading portion may also comprise a slot adapted to receive the proximal end of the coil. In one embodiment, the loading portion comprises a loading collar, and the tissue piecing structure can be rotated relative to the loading collar. Further, the tissue piercing structure can be moved axially relative to the loading collar to axially elongate the coil.

We further describe a method of closing a patent foramen ovale having a septum primum and a septum secundum and a tunnel extending therebetween. A countertraction element is positioned on one side of the patent foramen ovale. A closure device is delivered from the other side of the patent foramen ovale. The closure device is adapted to hold the septum primum and septum secundum together. The closure device is advanced into position while the countertraction element holds the position of at least one of the septa.

In one embodiment, positioning a countertraction element on one side of the patent foramen ovale comprises positioning the countertraction element in a left atrium of a patient. The countertraction element may comprise an expandable device such as a balloon, which may be delivered through the turmel of the patent foramen ovale or by penetrating through the septa of the patent foramen ovale. In another embodiment, the countertraction element comprises a cover releasably engageable with a delivery device, such as a guide wire or an anchoring element. In addition to providing countertraction, this cover can be secured to the septum at a patent foramen ovale, for example, with the closure device delivered through the cover. In another embodiment, the countertraction element includes a guide wire delivered through the turnnel to one side of the patent foramen ovale. The guide wire may have an S-shape, and one or more closure devices may be delivered through the septa of the patent foramen ovale adjacent the guide wire.

We also describe a method of closing an opening in a patient. A countertraction element is positioned relative to the opening to hold said opening in place. A closure device is delivered to the opening while the countertraction element holds the opening in place. In one embodiment, the opening may be a patent foramen ovale. In one embodiment, the countertraction element is a removable implant positioned within the opening.

### Brief Description of the Drawings

FIG. 1 is an anterior illustration of a heart, with the proximal parts of the great vessels.

FIG. 2 is a perspective view of a rotatable closure device in accordance with one embodiment of the present invention.

FIG. 2A is a perspective view of an alternative embodiment of a rotatable closure device in accordance with one embodiment of the present invention.

FIG. 3A is a perspective view of a closure device in accordance with one embodiment of the present invention.

FIG. 3B is an end view of the closure device of FIG. 3A.

FIG. 3C is a perspective view of an alternative embodiment of the closure device of FIG. 3A.

FIG. 4 is a schematic view of a closure device delivery system.

FIG. 5 is a detailed perspective view of the distal end of the closure device delivery system in accordance with one embodiment of the present invention.

FIG. 6 is a perspective view of a closure device delivery system in accordance with one embodiment of the present invention.

FIG. 7 is a perspective view of a loading collar used in the delivery system of FIG. 6.

FIGs. 8-13 are schematic views illustrating a closure procedure in accordance with one embodiment of the present invention.

FIG. 14 is a perspective view of a closure device delivery system adapted to deliver a plurality of closure devices.

FIGs. 15A and 15B are schematic views of a closure device delivered through a patent foramen ovale.

FIG. 16. is a schematic view illustrating a plurality of closure devices delivered through the septa of a patent foramen ovale.

FIGs. 17A and 17B are partial cross-sectional views of a closure device delivery system in accordance with another embodiment of the present invention.

FIG. 18 is a schematic view of a countertraction device to aid in delivery of a closure device.

FIGs. 19A and B are end views of the countertraction element of FIG. 18.

FIG. 19C is a perspective view of the countertraction element of FIG. 19A.

FIG. 19D is a perspective view of the countertraction element of FIG. 19B.

FIG. 19E is a perspective view of another embodiment of a countertraction element.

FIG. 20 is a schematic view of the countertraction device of FIG. 18 is a collapsed state.

FIG. 20A is a perspective view of the countertraction device of FIG. 20.

FIGs. 21A and B are schematic views of delivery of the countertraction device to a patent foramen ovale.

FIGs. 21C and D are schematic views of delivery of an implant to a patent foramen ovale with the countertraction device of FIG. 21B.

FIG. 22 is a schematic view of a countertraction device to aid in delivery of a closure device in accordance with an alternative embodiment of the present invention.

FIG. 22A is a detailed schematic view of the countertraction device of FIG. 22.

FIGs. 23A and B are cross-sectional views of a delivery system for delivering the countertraction devices of FIGs. 18 and 22.

FIG. 23C is a schematic view of a treatment site with a plurality of implants.

FIG. 24 is a schematic view of an expandable device positioned on one side of a patent foramen ovale acting as a countertraction element for delivery of a closure device.

FIG. 25A is a side view of one embodiment of a countertraction element having a cover releasably attached to a guide wire.

FIG. 25B is a side view of one embodiment of a countertraction element having a cover releasably attached to an anchoring element.

FIG. 25C is a perspective view of one embodiment of an anchoring element.

FIG. 26 is an end view of the countertraction element of FIG. 25A implanted at a patent foramen ovale.

FIG. 27 is a schematic view of the countertraction element of FIG. 26 implanted at a patent foramen ovale.

FIG. 28 is a schematic view of a guide wire acting as a countertraction element for delivery of a closure device.

FIGS. 29A and 29B are schematic views of an alternative embodiment of a guide wire acting as a countertraction element for delivery of a closure device through a catheter.

FIG. 30A is a schematic view of a removable implantable device acting as a countertraction element for delivery of a closure device.

FIG. 30B is an enlarged view of the implantable device of FIG. 30A.

### Detailed Description of the Preferred Embodiments

For simplicity, the embodiments of the present invention will be described primarily in the context of a patent foramen ovale closure procedure. However, the device herein is readily applicable to a wider variety of closure or attachment procedures, and all such applications are contemplated by the present inventors. For example, additional cardiac procedures such as atrial septal defect closure, ventricular septal defect closure, and atrial appendage closure are contemplated. Vascular procedures such as patent ductus arteriosis closure, isolation or repair of aneurysms, anastamosis of vessel to vessel or vessel to prosthetic tubular graft joints may also be accomplished using the devices as described herein. Attachment of implantable prostheses, such as attachment of the annulus of a prosthetic tissue, mechanical heart valve or an annuloplasty ring may be accomplished. A variety of other tissue openings, lumens, hollow organs and surgically created passageways may be closed in accordance with the preferred embodiments. Closures and repairs described herein may be accomplished using catheter based interventional methods or minimally invasive surgical methods. Adaptation of the devices disclosed herein to accomplish procedures such as the foregoing will be apparent to those of skill in the art in view of the disclosure herein.

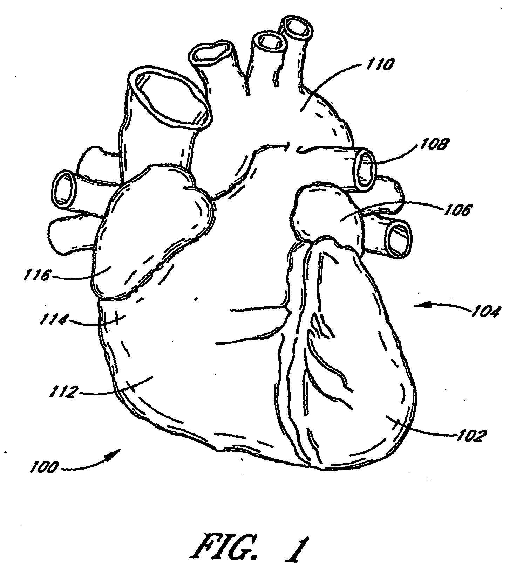

Referring to FIG. 1, a heart 100 is illustrated to show certain portions including the left ventricle 102, the left atrium 104, the left atrial appendage 106, the pulmonary artery 108, the aorta 110, the right ventricle 112, the right atrium 114, and the right atrial appendage 116. As is understood in the art, the left atrium 104 is located above the left ventricle 102 and the two are separated by the mitral valve (not illustrated).

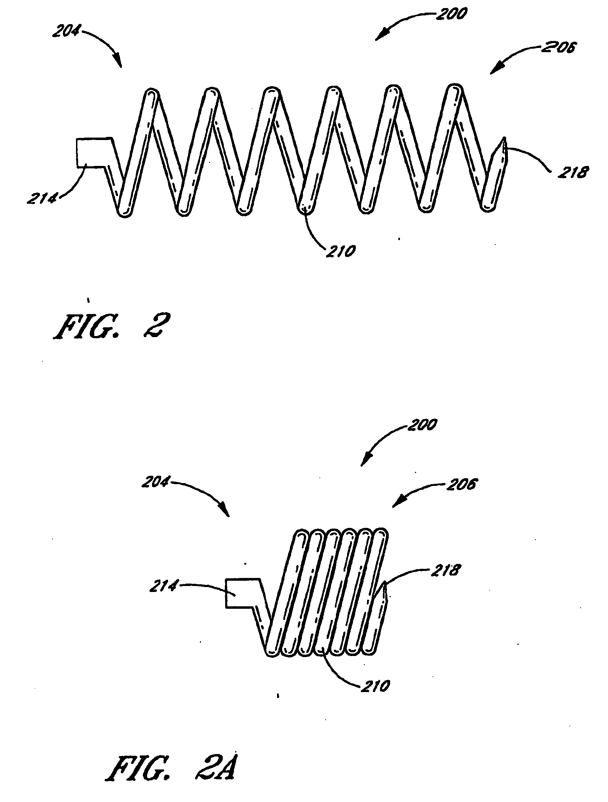

With reference to FIG. 2, a rotatable patent foramen ovale closure device is shown. In these embodiments, the patent foramen ovale is simply held together by positioning a device 200 to hold the septum primum and septum secundum together. The device 200 comprises a proximal end 204 and a distal end 206. The device has a spring-like configuration and comprises a coiled wire 210. The device 200 may include a detachment element 214 at its proximal end which may comprise a loop, internal threading, external threading, or other structures adapted to releasably engage the device 200 to a delivery device. The distal end 206 may include a sharpened point 218 for puncturing the tissue. The device may also be provided with a sleeve. FIG. 2A shows an embodiment of the device 200 wherein the closure device has a pitch less than the pitch shown in the closure device of FIG. 2.

In one embodiment, rotatable closure device 200 may have a left-handed threading. In another embodiment, rotatable closure device 200 may have a right-handed threading. In some embodiments, the coil 210 may have a variable pitch. In some embodiments, the diameter of the rotatable closure device may vary along the length of the device. In one embodiment, the coil has a diameter of about 1/8 to ½ inch.

To deliver the device 200, in one embodiment the proximal end 204 is positioned in the right atrium, while the distal end 206 is positioned in the left atrium, by rotating the device 200 through the septum secundum and septum primum using a delivery device releasably attached to the device 200 at detachment element 214. It is also envisioned that the proximal end 204 may be positioned in the left atrium, while the distal end 206 may be positioned in the right atrium, by rotating the device 200 through the septum primum and septum secundum. After delivery, the delivery device can be detached from detachment element 214. The device may be delivered such that after passing through the septum primum and septum secundum, the coil axially shortens due to its natural pitch, thereby pinching the septum primum and septum secundum together. In another embodiment, the coil may tend to axially shorten towards an unstressed shape due to the elasticity of the coil material.

Preferably, the device 200 is formed of a metal such as stainless steel, Nitinol, Elgiloy, or others which can be determined through routine experimentation by those of skill in the art. The wire may also be biodegradable. Wires having a circular or rectangular cross-section may be utilized depending upon the manufacturing technique. Wires may be stranded or cabled. In one embodiment, a circular coil made up of rectangular cross section wire is cut such as by known laser cutting techniques from tube stock. In another embodiment the wire coil is substantially formed on a coil winding machine. The closure device is preferably an integral structure, such as a single ribbon or wire, or element cut from a tube stock. In some embodiments the wire coil is made of Nitinol and heat set to a pre-determined shape which the coil tends to assume following coil deployment or implantation.

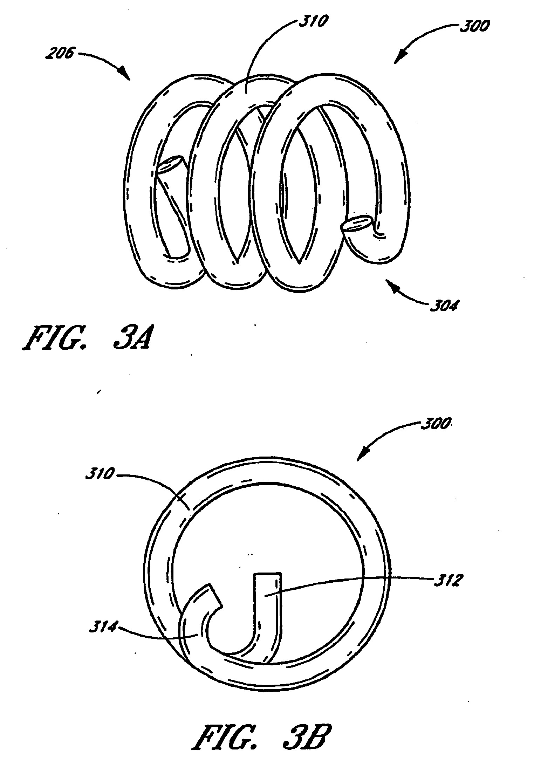

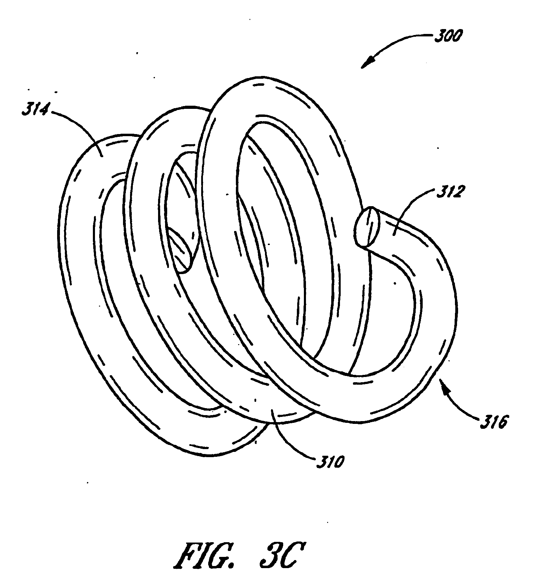

Figures 3A and 3B show another embodiment of a closure device in accordance with one embodiment of the present invention. The device 300 comprises a proximal end 304 and a distal end 306. The device may be a coil or have a helical spring-like configuration as described above, and as illustrated comprises a coiled wire 310. It will be appreciated that the term "coil" is a broad term and is used herein in its ordinary sense and includes, without limitation, coils, helical wires or ribbons, springs, or any other similarly shaped structure. The coiled wire is bent inward at its proximal end 304 and distal end 306 as shown in Figure 3B, to form a proximal tang 314 and distal tang 312, respectively. Figure 3C shows an embodiment of the device 300 wherein a bend 316 is provided in the coiled wire 310 near distal tang 312.

Examples of other coil shapes can be found in U.S. Patent Nos. 5,810,882 and 5,582,616, the entireties of which are hereby incorporated by reference.

In one embodiment, the closure device 300 may be made from a medical plastic or a metal, such as stainless steel, Nitinol, Elgiloy, polyester, PEEK or others which can be determined through routine experimentation by those of skill in the art. In another embodiment, the closure member 300 may be made of a dissolvable suture material. The closure device 300 may also be biodegradable. It is also envisioned that other metallic or non-metallic biocompatible materials may be used to form closure device 300. In one preferred embodiment, the closure device is a superelastic coil, which can be radially compressed and axially expanded from its natural, relaxed state to its stressed state.

In some embodiments, the closure device may be coated with a thin layer of a tissue ingrowth material, such as collagen, polyester, ceramic, and the like. The coating may be porous, such as a porous hydroxyapatite material. A Dacron, polyester, or other tissue growth prompting or accepting material may be used with the closure device. In one embodiment, at least a portion of the closure device may be coated with a fabric comprising the tissue ingrowth material. In one embodiment, the closure device may comprise a coating over at least a portion of the device. The closure device may be manufactured in any of a variety of ways, such as machining, molding, and the like.

The coiled wire 310 may have a circular, rectangular, or other shaped cross-section, depending upon the manufacturing technique. In one embodiment, a circular cross section is molded from a biocompatible polymer, such as polyethylene terephthalate (PET). In some embodiments, the closure device 300 may have any pitch or a variable pitch. In some embodiments, the diameter of the closure device may vary longitudinally.

For use in a patent foramen ovale, in one embodiment, the device 300 has an outer diameter D having any value or range of values from about 0.005 in to about 0.375 in, and, in one more preferred embodiment, about 0.11 in. The overall length L of the closure device 300 from the distal end 306 to the proximal end 304 in one embodiment is any value or range of values from about 0.040 to 0.120 in. In some embodiments, the wire has a diameter of any value or range of values between about 0.005-0.02 in, and in some preferred embodiments, any value or range of values between about 0.008-0.014 in, and in one more preferred embodiment, about 0.010 in.

In some embodiments, radiopaque markers may be provided on the closure device 300 to aid in placement at the treatment site. In some embodiments, the radiopaque markers are crimped on to the closure device. In one embodiment, the radiopaque markers are tubular bands crimped on to the closure device. In some embodiments, the radiopaque markers are coatings applied to the device. In some embodiments, the radiopaque markers may be platinum or iridium, and the like. In some embodiments the radiopaque marker is a wire core, wire coating, or wire strand of radiopaque material.

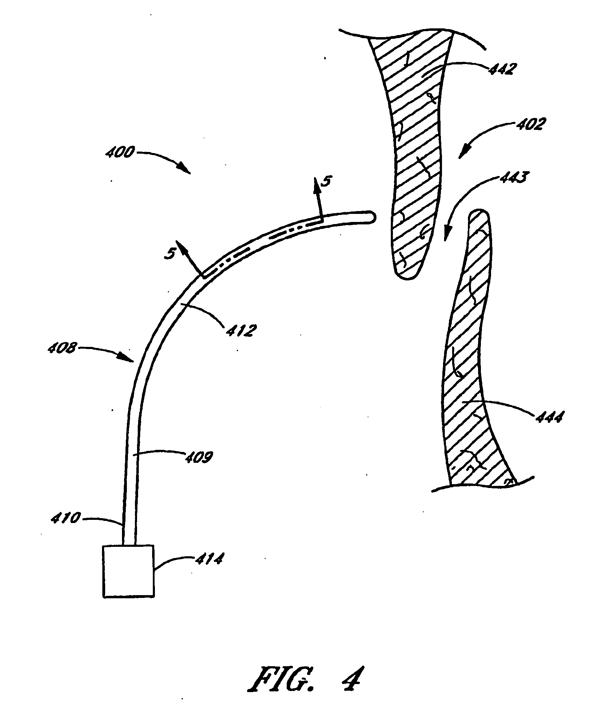

Referring to Figure 4, a delivery device 400 may be used to deliver the closure device to the patent foramen ovale 402. The patent foramen ovale 402 generally includes a septum primum 444 and a septum secundum 442 and a tunnel 443 extending therethrough. The delivery device 400 comprises a catheter 408 having an elongate flexible tubular body 409 extending between a proximal end 410 and a distal end 412. The catheter is shown in a highly schematic form, for the purpose of illustrating the functional aspects thereof. The catheter body will have a sufficient length and diameter to permit percutaneous entry into the vascular system, and transluminal advancement through the vascular system to the desired deployment site. For example, in an embodiment intended for access at the femoral artery and deployment within the right atrium, the catheter 408 will have a length within the range of from about 50 cm to about 150 cm, and a diameter of generally no more than about 15 French. Further dimensions and physical characteristics of catheters for navigation to particular sites within the body are well understood in the art and will not be further described herein.

The flexible body can be manufactured in accordance with any of a variety of known techniques. In one embodiment, the flexible body 409 is extruded from any of a variety of materials such as HDPE, PEBAX, nylon, and PEEK. Alternatively, at least a portion of or all of the length of the tubular body may comprise a spring coil, solid walled hypodermic needle or other metal tubing, or a braided reinforced wall, as are known in the art. The spring coil, tubing, braided reinforcement, or other structures may be encapsulated with thermoset polymers such as polyimide or with thermoplastic polymers such as PEBAX, and the like.

The tubular body 409 may be provided with a handle 414 generally on the proximal end 410 of the catheter 408. The handle 414 may be provided with a plurality of access ports. The handle 414 may be provided with an access port which may be used as a guide wire port in an over the wire embodiment, and a deployment wire port. Additional access ports, such as a contrast media introduction port, or others may be provided as needed, depending upon the functional requirements of the catheter. The catheter 408 may be constructed to contain the same number of ports as the handle 414. The handle 414 permits manipulation of the various aspects of the occlusion device delivery system 400, as will be discussed below. Handle 414 may be manufactured in any of a variety of ways, typically by injection molding, machining or otherwise forming a handpiece for single-hand operation, using materials and construction techniques well known in the medical device arts.

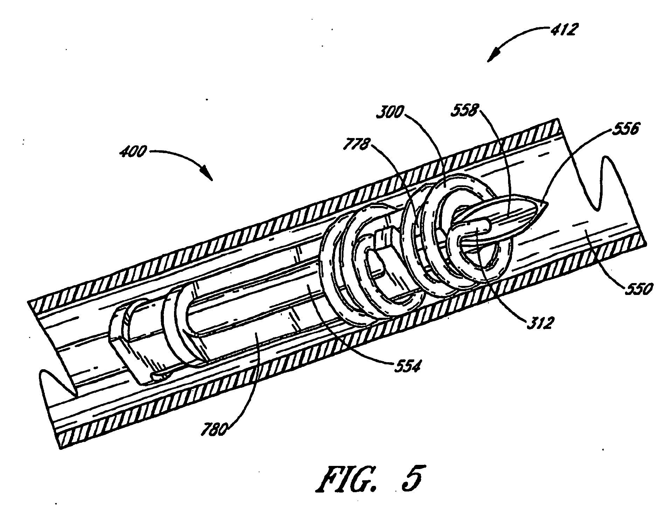

With reference to Figure 5, the catheter 408 may include a passageway 550 for delivery of the closure device 300 to the patent foramen ovale. An elongate body may be delivered through the passageway 550, and may include a tissue piercing structure 554, such as a needle. The tissue piercing structure 554 may have a generally tubular body and is moveable axially and rotationally relative to the catheter 408. In one embodiment, the tissue piercing structure may be spring-loaded to advance upon actuation to a location distal of the distal end 412 of the catheter. The tissue piercing structure 554 may have a pointed end 556 for accessing the patent foramen ovale, as will be described below. At the distal end of the tissue piercing structure 554, an opening 558 may be provided in which the distal end 306 of the closure device 300 engages. More preferably, the distal tang 312 of the closure device 300 engages with opening 558. As illustrated in Figure 5, a loading portion for the proximal tang 314 is provided in the form of a loading collar 778 over the tissue piercing structure 554. Loading collar 778 includes an opening 780, more preferably a longitudinal slot, which releasably engages the proximal end 304 of the closure device, more preferably engaging proximal tang 314.

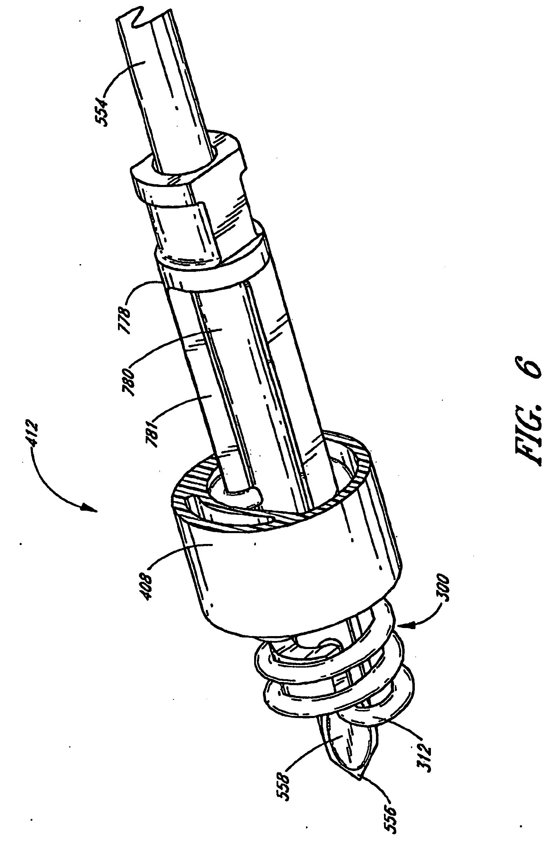

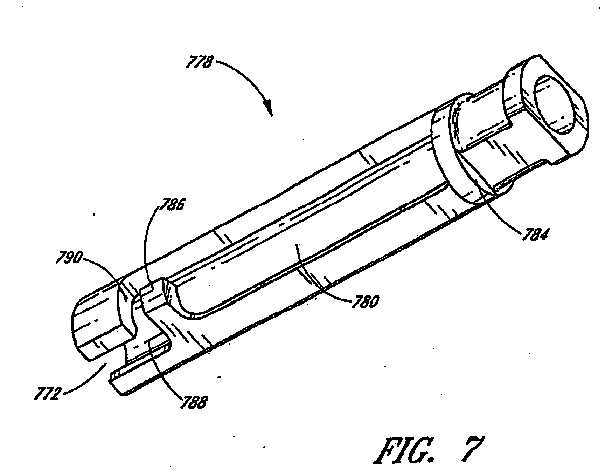

Figures 6 and 7 illustrate in perspective view one embodiment of a tissue piercing structure 554 with a loading collar 778 provided thereover. As shown in Figure 6, the tissue piercing structure 554 may have a pointed end 556 for accessing the patent foramen ovale, and has an opening 558 for receiving distal tang 312 of the closure device 300. The tissue piercing structure 554 may be axially slideable and rotatable relative to loading collar 778, which releasably engages a proximal tang 314 as described above. The catheter 408 is illustrated in a partially cut-away view, having a plurality of lumens extending therethrough.

Loading collar 778 is preferably an elongate tubular body which may extend to the proximal end of catheter 408, or may be operated from the handle 414 using a suitable actuator extending through the catheter 408. For example, the loading collar may include a proximal flexible section that extends to the proximal end of the catheter 408. In one embodiment, the loading collar is rotatable about ¼ of a turn relative to the tissue piercing structure.

An elongate opening or slot 780 extends longitudinally along the loading collar, and as shown in Figure 7, includes a projection 786 toward a distal end thereof, near the pointed end of the tissue piercing structure. The opening 780 and projection 786 form a track 788 which is used to guide and release the proximal tang 314 (as well as the distal tang 312 in the multiple coil embodiment, described below) of the closure device 300 from the loading collar 778 for deployment of the closure device, as described below. The track 788 is shown having a generally vertical portion 790 which is generally parallel to the projection 786 and a generally horizontal portion 792 which is generally perpendicular to the vertical portion 790 and parallel to the longitudinal axis of the opening 780. The horizontal portion 792 of the track 788 extends to the distal end of the loading collar 778.

In some embodiments, as shown in Figure 6, the closure device 300 is loaded by engaging distal tang 312 with the opening 558 on tissue piercing structure 554, and wrapping the turns of the coils of closure device 300 over the loading collar 778, which extends proximally from the opening 558. The proximal tang 314 engages opening 780 in loading collar 778 proximal to projection 786. Rotating the tissue piercing structure 554 relative to the loading collar 778, for example clockwise (for a clockwise wound coil when viewed from the proximal end of the device), and axially advancing the tissue piercing structure 554 relative to the loading collar 778, causes the distal tang to rotate and move distally away from the projection 786. With continued clockwise rotation of the tissue piercing structure 554 relative to the loading collar 778, the long wall 781 of the opening 780 engages the tang 314 to radially compress the device 300 around the cylindrical body of the tissue piercing structure 554. The device loaded in this configuration may be advanced through tissue, as described below.

To release the closure device 300 from the tissue piercing structure 554 and loading collar 778, the tissue piercing structure 554 may be rotated, for example counter-clockwise, thereby allowing the coil to achieve its natural diameter in the tissue. The tissue piercing structure may then be axially retracted relative to the loading collar 778, pulling the tissue piercing structure from the tissue, as described below, and proximally through the device 300. Finally, the loading collar 778 may be rotated, for example clockwise, until the proximal tang 314 snaps out of the track 788, sliding along the vertical portion 790 and out horizontal portion 792.

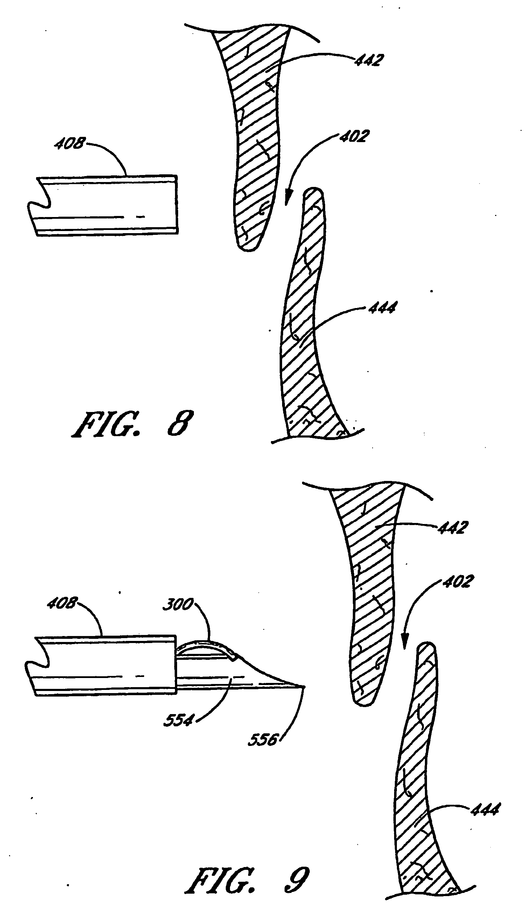

A method of delivering the closure device 300 to a treatment site, such as a patent foramen ovale, is shown in Figures 8-13. In use, the delivery device 400 is percutaneously introduced into the vascular system and transluminally advanced into the heart and, subsequently, to the patent foramen ovale using techniques which are known in the art.

The patent foramen ovale may be accessed via catheter through a variety of pathways. In one embodiment, the patent foramen ovale may be accessed from the venous circuit. The catheter may be introduced into the venous system, advanced into the inferior vena cava or superior vena cava and guided into the right atrium. The catheter may then be directed to the patent foramen ovale. Alternatively, once in the right atrium, the catheter may be advanced through the tricuspid valve and into the right ventricle and directed to a ventricular septal defect and the closure device deployed.

Alternatively, the patent foramen ovale may be accessed from the arterial circuit. The catheter is introduced into the arterial vascular system and guided up the descending thoracic and/or abdominal aorta. The catheter may then be advanced into the left ventricle through the aortic outflow tract. Once in the left ventricle, the catheter may be directed up through the mitral valve and into the left atrium. When the catheter is in the left atrium, it may be directed into the patent foramen ovale and the closure device deployed.

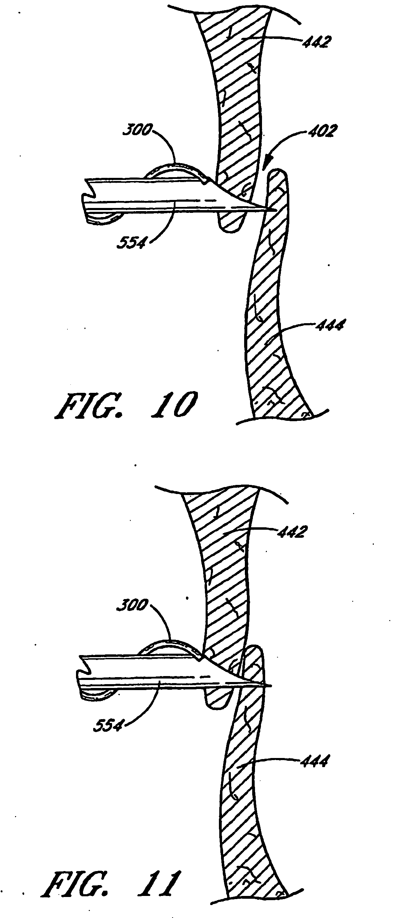

As shown in Figure 8, a catheter 408 approaches a patent foramen ovale from the right atrium. Initially, the closure device 300 is configured inside the catheter 408. As shown in Figure 9, the tissue piercing structure 554, which releasably engages distal tang 312 of closure device 300, is advanced out of the catheter 408. Loading collar 778 (not shown in Figure 9) may also be advanced out of the catheter 408 with the tissue piercing structure. With the closure device 300 releasably engaging the tissue piercing structure 554, the tissue piercing structure 554 may be advanced and rotated (for example, about ¼ of a turn) relative to the loading collar, to stretch and radially compress the closure device 300, as described above. The tissue piercing structure is advanced through the septum secundum 442 and septum primum 444, as shown in Figures 10-12. In some embodiments, tissue piercing structure 554 may be advanced across the septa manually. In other embodiments, tissue piercing structure 554 may be advanced across the septum using a spring loaded handle. In one embodiment, while penetrating the septa the tissue piercing structure and loading collar are also rotated to assist in penetration.

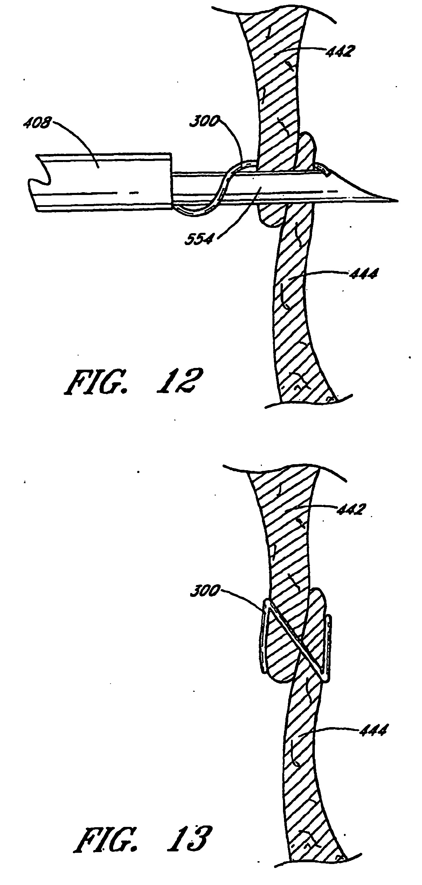

In one embodiment, the tissue piercing structure is advanced such that at least one helical section of the closure device crosses the septa. After optimal positioning is achieved, the closure device 300 can be released and the catheter 408 can be removed, as shown in Figure 12. Release of the closure device 300 may occur, for example, by proximally retracting the tissue piercing structure 554, as described above. Rotation of the tissue piercing structure 554, relative to the loading collar 778, allows the implant 300 to relax towards its natural diameter. Retraction of the tissue piercing structure 554 past the septum primum and septum secundum allows the distal tang 312 to exit the opening 558 of the tissue piercing structure. Finally, rotation of the loading collar relative to the implant allows the proximal end of the closure device 300 to snap out of the loading collar 778, releasing the device 300. When release is complete, the closure device should be in or biased towards its natural state, preferably radially expanded and axially contracted. This allows the closure device to pinch together the septum primum and septum secundum to close the patent foramen ovale. This thereby results in a continual closure force on both the septum primum and septum secundum.

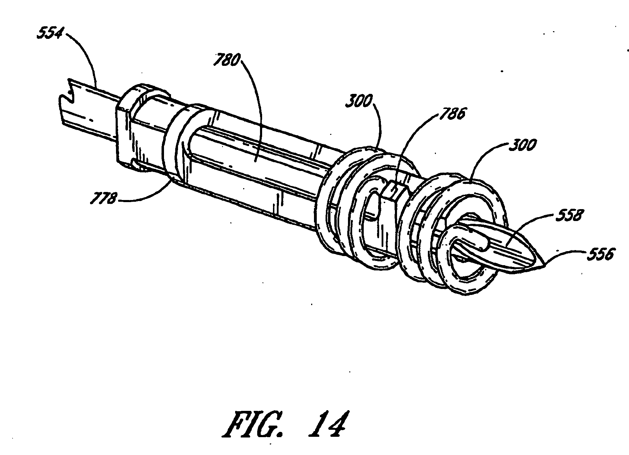

In one embodiment, multiple closure devices may be delivered during the same procedure by providing two or more closure devices on the loading collar. Any number of closure devices may be delivered in this manner. Figure 14 shows loading collar 778 having a plurality of closure devices 300 provided thereon. The distal most device 300 is loaded onto the tissue piercing structure 554 and loading collar 778 as described with respect to Figures 6 and 7 above. Additional closure devices are provided on the loading collar 778, proximal of the projection 786, with proximal tang 314 and distal tang 312 of each device extending into the opening 780.

After delivery of the distal most device 300 as described above, the tissue piercing structure 554 can be retracted proximally into the loading collar 778 until the distal tang 312 of the next device 300 snaps into the opening 558 of the tissue piecing structure. The tissue piercing structure is then advanced, pulling the device 300 along the open slot 780 of the loading collar 778. By rotating and advancing the tissue piercing structure 554, the distal tang is guided through the track 788 until the device again sits at the distal end of the loading collar. The device is then deployed as previously described.

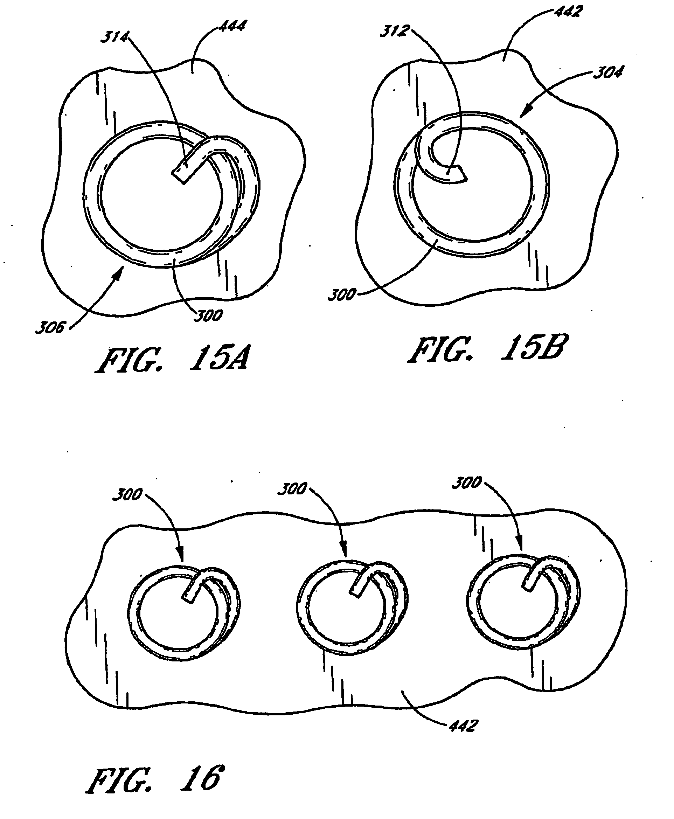

Figure 15A shows the distal end 306 of the closure device 300 delivered at the septum primum 444 and Figure 15B shows the proximal end 304 of the closure device 300 delivered at the septurn secundum 442. Material exposure in both the right and, more importantly, the left atria is minimal, thereby reducing the risk of clot formation. Figure 16 shows an embodiment wherein a plurality of closure devices 300 are delivered to the septal defect, such as by using the devices described above. As shown in Figure 16, three closure devices 300 extend through the septa of the patent foramen ovale, each adjacent to one another. Although the closure devices of Figure 16 are shown in a linear arrangement, it will be appreciated that other configurations may be used to adequately close the patent foramen ovale. In one embodiment, closure devices may be first deployed at the corners of the mouth of the patent foramen ovale, and then across the tunnel of the patent foramen ovale.

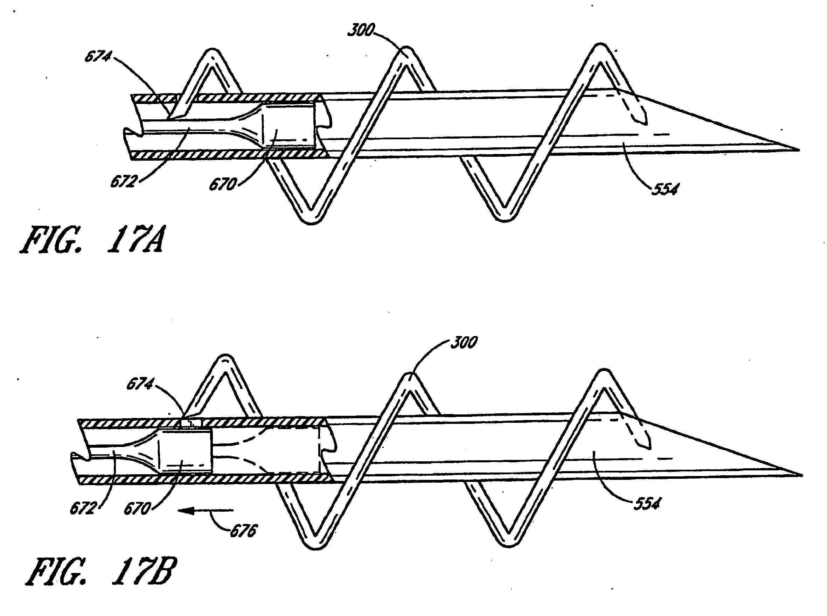

Figure 17A and 17B illustrate another embodiment of a delivery device used to deliver a closure device 300 such as described above. A tissue piercing structure 554 is provided similar to that described above, having an opening 558 for releasably engaging a distal tang 312 of closure device 300. Tissue piercing structure 554 also includes a loading portion, which may include an opening 674 proximal to the opening 558 for releasably engaging a proximal tang 314 of closure device 300. As illustrated, the loading portion is integral with the tissue piercing structure, although in another embodiment, it may be formed from a separate piece. An actuator 672 extends through the lumen of the tissue piercing structure, and preferably includes a release element 670 at a distal end thereof. When the actuator 672 and release element 670 are actuated in the direction of arrow 676, as shown in Figure 17B, the proximal end of the closure device 300 is pressed out of the opening 674 and tissue piercing structure 554. In some embodiments, the actuator and release element may be actuated in the direction opposite the direction of arrow 676 to release the closure device 300 from the tissue piercing structure 554.

In some embodiments, the closure device 300 may be stretched out along the length of the tissue piercing structure 554 and may be rotated axially, thereby reducing the diameter of the closure device. The closure device may be delivered in a manner similar to that described above, wherein the tissue piercing element 558 penetrates the septa of a patent foramen ovale. Then, the release element 670 may be actuated to release the closure device 300, and the tissue piercing element can be proximally retracted. The closure device upon being released returns to its natural state, pinching the septa of the patent foramen ovale together.

In some embodiments, radiopaque markers may be provided on the tissue piercing structure 554 or delivery device 400 to aid in placement at the treatment site. In some embodiments, the radiopaque markers are crimped on to the tissue piercing structure or delivery device. In one embodiment, the radiopaque markers are tubular bands crimped on to the tissue piercing structure of delivery device. In some embodiments, the radiopaque markers are coatings applied to the structure or device. In some embodiments, the radiopaque markers may be platinum or iridium, and the like.

### Countertraction Elements

In another embodiment, the delivery device 400 such as catheter 408 may be provided with an opening for receiving a countertraction element. The opening may be the same opening as that used to deliver a closure device, or may be a separate lumen in the catheter 408. For example, as shown in Figure 4 above, one of the additional openings in the catheter 408 may be used to deliver the countertraction element. Alternatively, the countertraction element may be delivered using a separate delivery device.

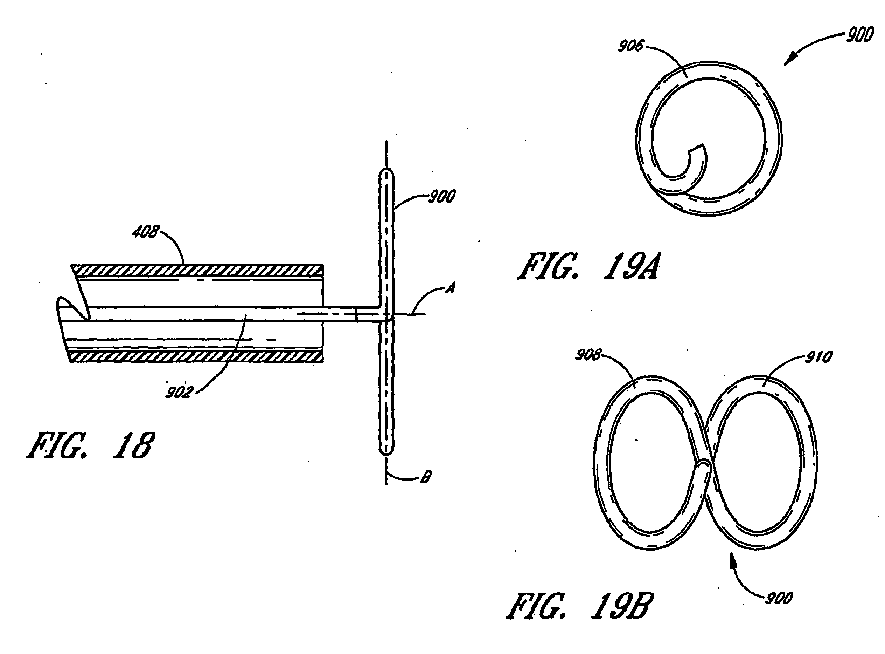

Figure 18 is directed to an embodiment having an expandable countertraction element 900. The expandable countertraction element 900 may be integral with or releasably attached to a guide wire 902 or other elongate body which is slideably received within a catheter 408. In some embodiments when the countertraction element is in its expanded state, an axis B extending through the countertraction element 900 is generally transverse to the longitudinal axis A of the guide wire 902. In one embodiment, the countertraction element 900 is a substantially planar device, the plane of the device being parallel to axis B when the device is expanded.

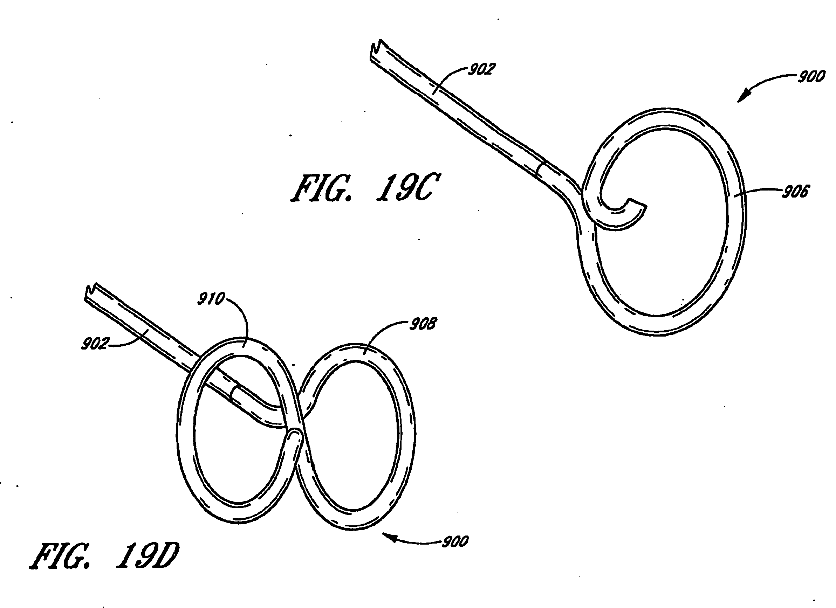

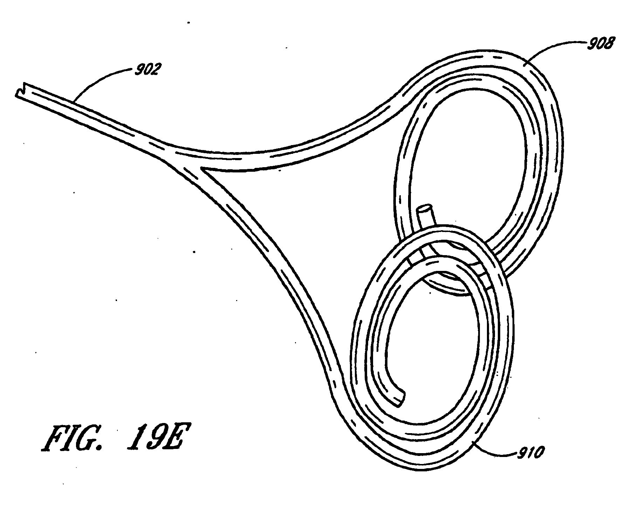

The expandable countertraction element 900 in one embodiment may be a wire element. In one embodiment, the expandable countertraction element 900 in its expanded state is a circular loop 906, as shown in Figure 19A. In one embodiment, the expandable countertraction element 900 in its expanded state includes two loops 908, 910, as shown in Figure 19B. Figures 19C and 19D are perspective views of the embodiments shown in Figures 19A and 19B, respectively. It will be appreciated that the expandable countertraction element may have any suitable shape. Figure 19E illustrates another embodiment of a countertraction element which includes a wire 902 that divides in its distal end into two loops 908 and 910. In this embodiment, the loops 908 and 910 generally lie in a plane parallel to the axis of the longitudinal axis of the wire 902. Other suitable shapes include, but are not limited to, oval, paper-clip shape, triangular, rectangular, a flower-petal shape, and the like. In some embodiments, the countertraction element and guide wire is a shape memory or superelastic material. In one embodiment, the shape memory material is Nitinol. Other shape memory alloys or other metal alloys may also be used.

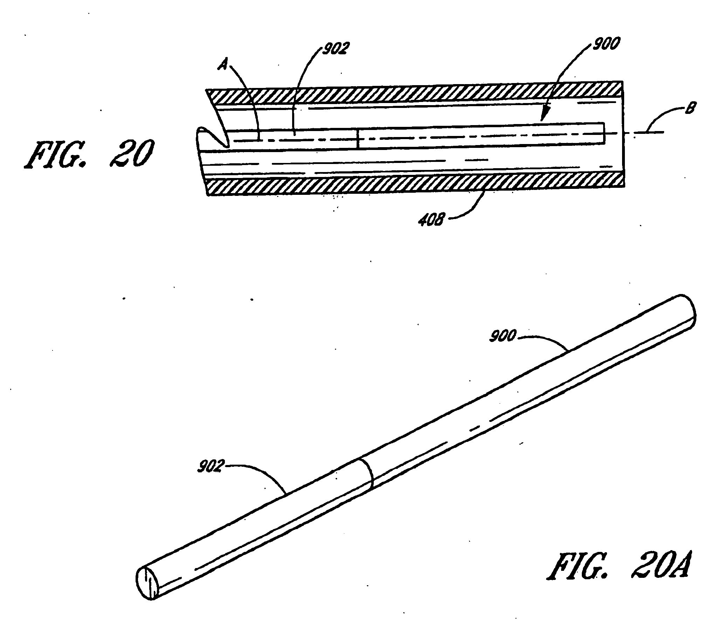

Figures 20 and 20A show the countertraction element 900 in its pre-expanded state. The countertraction element unwinds as it is retracted into the catheter, such that the axis B of the countertraction element is substantially collinear with the axis A of the guide wire. Similarly, when the guide wire is advanced out of the catheter, the countertraction element self-expands into its natural state.

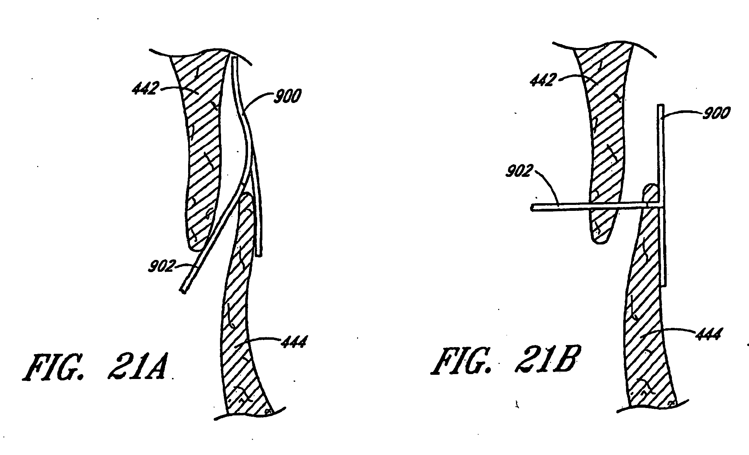

Figures 21A and 21B show embodiments of the countertraction element delivered at a treatment site. Figure 21A shows the countertraction element 900 positioned next to the septurn secundum 442 and septurn primum 444, and the guide wire 902 positioned between the septurn secundum 442 and septum primum 444. In this embodiment, the catheter 408 may be delivered through the tunnel of the patent foramen ovale between the septum primum and septum secundum. The guide wire 902 may be advanced to allow the countertraction element 900 to exit the distal end of catheter 408 and self-expand in the left atrium. In some embodiments, proximal traction on guide wire 902 assures contact between the countertraction element 900 and the septa 442 and 444. Figure 21B shows the countertraction element 900 positioned against the septum primum 444, and the guide wire 902 and countertraction element 900 punctured through the septum secundum 442 and septum primum 444. In one embodiment, a distal end of the countertraction element 900 may be sufficiently sharp to penetrate the septa. Alternatively, a hollow tissue piercing structure (not shown) may be provided to pierce the septa, and the guide wire 902 and countertraction element 900 is delivered through the hollow tissue piercing structure. In some embodiments, proximal traction on guide wire 902 assures contact between the countertraction element 900 and the septa 442 and 444.

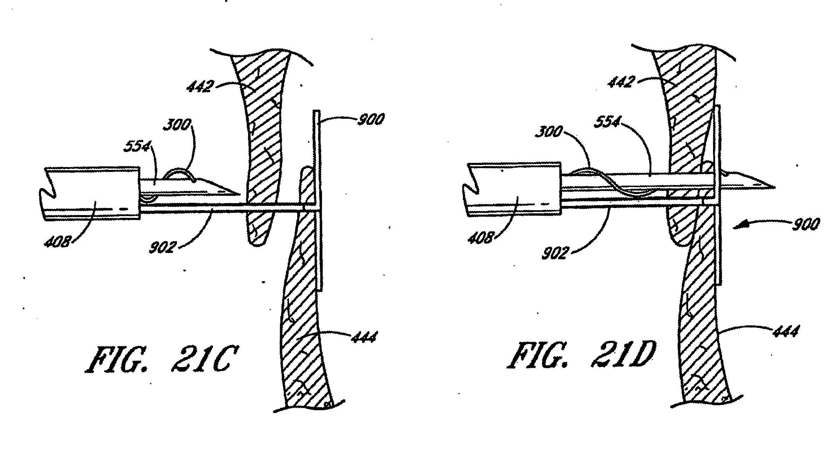

Figures 21C and D show delivery of an implant to a patent foramen ovale when the countertraction element is positioned in its expanded state at the patent foramen ovale. The countertraction element may be delivered using the methods described with respect to Figure 21A or Figure 21B. The implant is then delivered using a method as described with reference to Figures 8-13. The countertraction element is designed such that there is no interference between delivery and placement of the implant and the countertraction element.

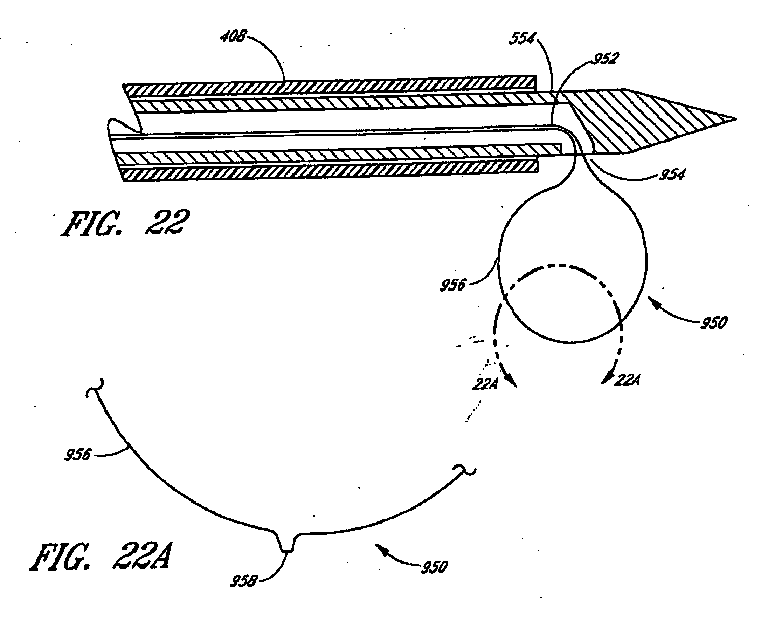

Figure 22 shows an embodiment where a countertraction element 950 is delivered within a tissue piercing structure 554, such as described hereinbefore with respect to Figure 5. The tissue piercing structure 554 may be provided with an elongate opening 952, which terminates in a distal port 954, in which the countertraction element is slideably received. The countertraction element 950 in one embodiment comprises a snare-like element 956. In one embodiment, the countertraction element is similar to the snare described in U.S. Patent No. 5,171,233, herein incorporated by reference. The countertraction element may be made of a solid Nitinol wire, stranded Nitinol wire, cabled Nitinol wire. The countertraction element may have other suitable structures, or be made of other materials including metals such as stainless steel, or engineering polymers, such as polyimide, PEEK, and the like. The countertraction element may be made of any material which is sufficiently rigid so that it can be pushed out of the port, but sufficiently flexible so that it can be collapsed within the opening.

In some embodiments, the countertraction element may include a beak 958 to facilitate folding of the countertraction element through the port of the tissue piercing structure. See Figure 22A. Beak 958 may be a partial loop (as shown), a loop 270 degrees or more in circumference, or may be comprised of a locally thinned out cross-section of snare-like element 956, or other suitable structure.

The tissue piercing structure is advanceable through a catheter 408, as described above. The countertraction element 950 is delivered to the treatment site with the tissue piercing structure withdrawn completely into the catheter 408 and countertraction element 950 completely withdrawn into the tissue piercing structure. When treating a patent foramen ovale, in one embodiment the catheter 408 is positioned adjacent the patent foramen ovale, and the tissue piercing structure 554 is advanced out of the catheter and across the septa until the port is distal the septum primum and septum secundum. In some embodiments, the tissue piercing structure 554 pierces both septa. In some embodiments, the tissue piercing structure is advanced between the septum primum and septum secundum through the PFO tunnel. The position of the port can be confirmed by injection of radiopaque dye contrast media through the tissue piercing structure. The countertraction element is advanced out of the port until the countertraction element is fully deployed. The countertraction element can be at least partially radiopaque to help assure that the countertraction element is fully deployed. Radiopaque dye contrast media or echo may also be used to ensure no interference between the closure device delivery system and the countertraction element.

The tissue piercing structure and the countertraction element are withdrawn slightly proximally to assure firm contact of the countertraction element with the septum primum. When the septa are sufficiently countertracted, in one embodiment, the catheter 408 and tissue piercing structure 554 are removed, and the implant may be delivered to the treatment site, for example, with a separate catheter and tissue piercing structure. The implant may be delivered using the methods as described with reference to Figures 21C and 21D. Preferably, the ring or diameter of the snare is sufficiently large so that the closure device is delivered inside the snare, and does not contact it. Furthermore, the countertraction element is preferably made of a sufficiently strong wire or ribbon that the delivery system for the implant does not damage it. The at least partially radiopaque countertraction element can be used as a target for septal puncture with an implant device. The countertraction element may be removed after delivery of the implant.

In one embodiment, the same tissue piercing structure may be used to deliver the countertraction element and closure device. In such an embodiment, the countertraction element may have a shepherd's crook shape or a distal hook to engage the septum and hook over the septum primum. The countertraction element in this embodiment would hold the septum while the closure device is advanced a sufficient distance into and past the septum primum. When the closure device is a coil, this distance allows the coil to shorten and expand to its natural state to sufficiently pinch and hold the septa together.

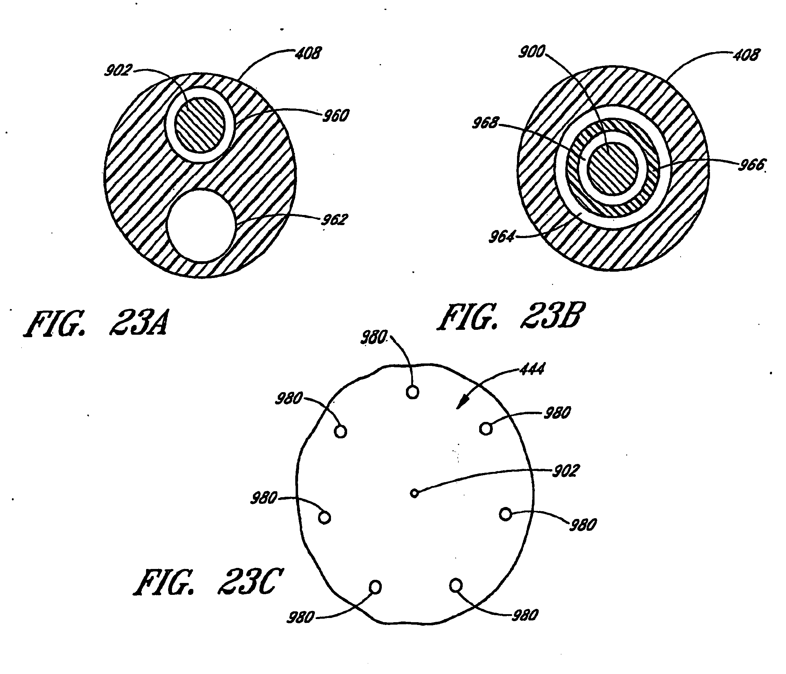

Figures 23A and 23B show a cross-sectional view of a catheter used to deliver the implant and countertraction system together. In one embodiment, as shown in Figure 23A, the catheter comprises a first lumen 960 for slideably receiving the countertraction element 900, and a second lumen 962 for slideably receiving an implant. Alternatively, as shown in Figure 23B, the catheter comprises an over-the-wire system having a lumen 964. An implant delivery system 966, slideably receivable within catheter lumen 964 also comprises a lumen 968, which slideably receives the countertraction element 900. In the over-the-wire embodiment, the countertraction element may, in some embodiments, exit the catheter a distance proximal to the end of the closure device delivery system, such that the tissue piercing structure pierces the septum secundum just above the entrance of the patent foramen ovale.

In one embodiment, the catheter of Figure 23A may be rotated or repositioned at the treatment site about the longitudinal axis A defined by guide wire 902, such that multiple implants can be delivered through lumen 962. This step can be repeated several times to enable placement of a number of implants in a generally circular array around the location of guide wire 902, as shown in Figure 23C.

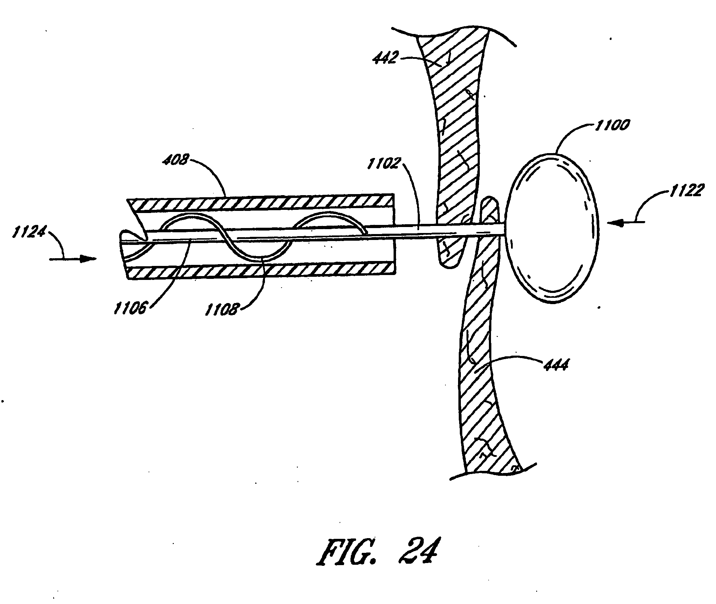

Figure 24 is directed to an embodiment of a device having an expandable countertraction element 1100. The expandable countertraction element 1100 may be provided on a guide wire 1102 or other elongate body which is slideably received within a catheter 408. In some embodiments, the guide wire may slide within an inner catheter 408 which may also include an implantable device 1108 for securing the patent foramen ovale in a closed position, such as closure device 300 described above. In some other embodiments, the guide wire carrying the countertraction element is coaxial with the device carrying the implantable closure device 1108.

The expandable countertraction element 1100 in one embodiment may be an inflatable balloon. In other embodiments, the expandable countertraction element 1100 may be an expanding wire structure, a braided expanding structure, a spoked expanding structure, frame, or the like. In some embodiments, the expanding spoked structure is laser cut from hypotube. In some embodiments, the inflatable balloon may be reinforced. Reinforcement of the balloon may prevent puncturing of the balloon by the implant. In some embodiments, the expandable countertraction element is self-expanding. In other embodiments, the expandable countertraction element is actively expanded. In one actively expandable embodiment, the system is a push-pull system having a hypotube which slideably receives a wire. The hypotube is connected to the proximal end of the countertraction element, and the wire is connected to the distal end of the countertraction element. The wire is retracted while advancing the hypotube, thereby expanding the countertraction element.

In one embodiment, the expandable countertraction element may be a long, thin balloon structure adapted for delivery through the tunnel of the patent foramen ovale. For example, the catheter 408 may be delivered to the heart, such as described above, and into the right atrium as illustrated in Figure 24. The balloon may be advanced to the other side of the patent foramen ovale, either by passing through the tunnel of the patent foramen ovale, or by penetrating through the septa of the patent foramen ovale, as illustrated in Figure 24. For example, the balloon may be contained within a separate delivery catheter (not shown) which may have a sharpened end for penetrating the septa, or may have a flat configuration to more easily pass through the tunnel of the patent foramen ovale.

Once the countertraction element 1100 is positioned on the other side of the patent foramen ovale (as illustrated in Figure 24, adjacent the septum primum 444 in the left atrium), the countertraction element may be expanded, such as by inflating the balloon or by manipulating a suitable actuator to deploy the countertraction element. The expandable countertraction element 1100 is drawn toward the septum primum 444 in the direction of arrow 1122 and the implantable closure device 1108 is delivered through the septa of the patent foramen ovale. Because the countertraction element 1100 provides a proximal retaining force against the septum primum 444, while the closure device 1108 is advanced through the septum secundum 442 and septum primum 444, the two septa are held in position relative to one another. The catheter 408 may also be repositioned to select a desirable implantation site for the closure device 1108, such as described above. Once the device is in position, the countertraction element 1100 can be collapsed or deflated, and the countertraction element 1100 can be withdrawn, for example, proximally through the closure device 408. Optionally, additional devices may be delivered while the countertraction element is in position.

Although Figure 24 illustrates the closure device 1108 being delivered over the same elongate body 1102 as that carrying the balloon 1100, it will be appreciated that a separate device may be used to provide countertraction during delivery of the closure device. A separate delivery catheter may be desirable, for example, to selectively implant multiple closure devices at desired locations. Moreover, any number of suitable closure devices may be delivered while using the countertraction element. Further details of other closure devices may be found in published Application Serial No 2004 0220596, filed February 4, 2004, entitled "Patent Foramen Ovale Closure System"; Application Serial No. 2005 018 7568, filed February 20, 2004, entitled "Devices and Methods for Closing a Patent Foramen Ovale with a Coil-Shaped Closure Device"; and U.S. Publication No. 2003-0028213 A1, the entirety of which are hereby incorporated by reference.

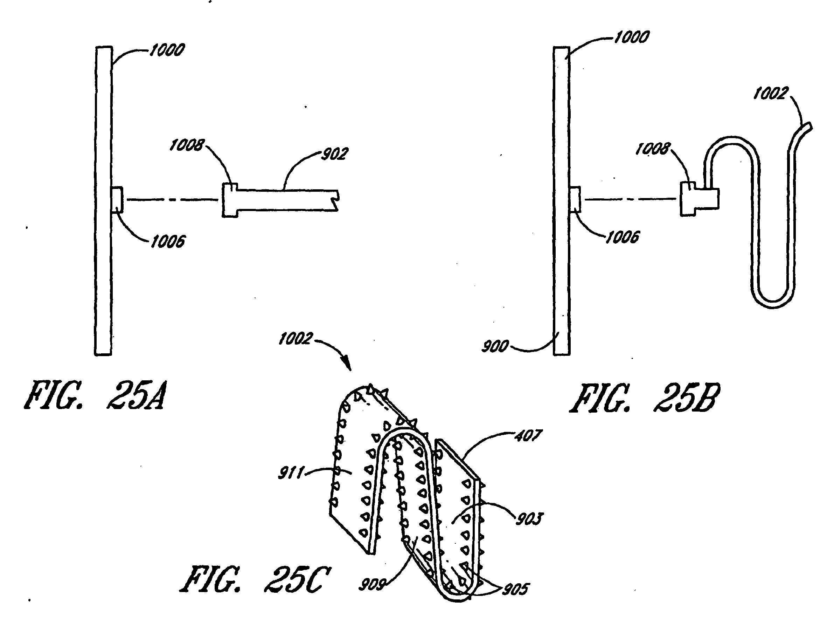

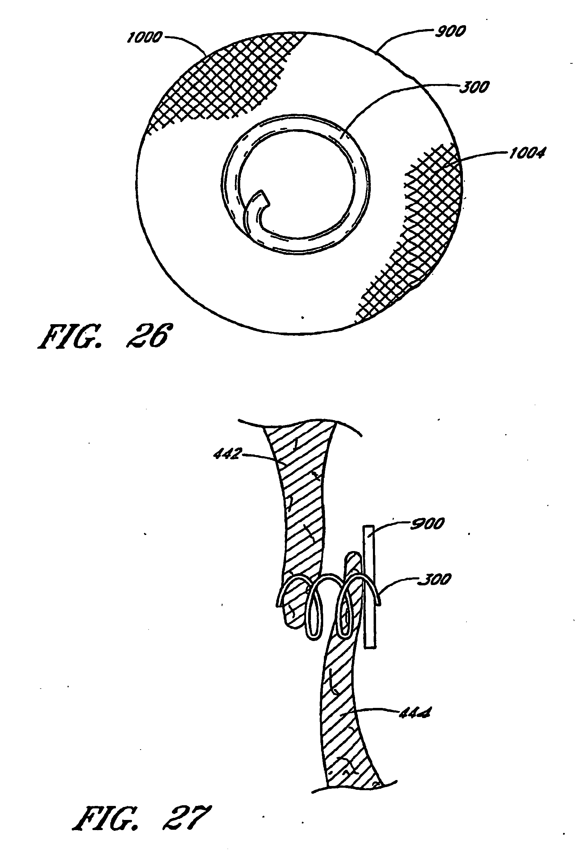

Figures 25A-27 show another embodiment of a device having a countertraction element 1000. The countertraction element may be similar to the countertraction elements described in Figures 18-19D above, and may comprise a wire element 900 having a loop, ring or snare-like shape. As shown in Figure 26, the countertraction element preferably includes a cover extending over the wire. As illustrated in Figure 26, the cover may be generally circular, or have any other suitable shape. The cover may be made of any suitable material, including a metal, polymer, or biocompatible material, and may comprise a mesh, solid sheet of material, or other suitable structure.

In one embodiment, the countertraction element 1000 is releasably connectable to a guide wire 902 at attachment elements 1006, 1008. Attachment elements may include snap fittings, threading or any other suitable mechanism to releasably attach the countertraction element and guide wire together. Alternatively, the countertraction element can be releasably or permanently attached to an anchoring element 1002, as shown in Figure 25B, which may itself may be releasably attached to a guide wire 902 (not shown). The anchoring element 1002 as illustrated is preferably superelastic and has a clip shape adapted to engage over the septum primum and septum secundum.

As shown in Figure 25C, the clip comprises a body 903 having at least one anchor 905. The body 903 comprises a proximal section 907, an intermediate section 909, and a distal section 911. The proximal section 907 in one embodiment is adapted to be positioned in the right atrium, the distal section 911 is adapted to be positioned in the left atrium, and the intermediate section 909 is adapted to be positioned between the septum primum and septum secundum in the tunnel of the patent foramen ovale. In the illustrated embodiment, a plurality of barbs or other anchors 905 are provided, for engaging adjacent tissue to retain the anchoring clip 1002 in its implanted position and to limit relative movement between the tissue and the device. The illustrated anchors are provided on at least one side of the body 903, and on at least one section (proximal, distal, intermediate). Preferably, each section is provided with one or two or more anchors each. Any of a wide variety of structures may be utilized for anchors 905, such as hooks, barbs, pins, sutures, adhesives, ingrowth surfaces and others which will be apparent to those of skill in the art in view of the disclosure herein.

In some embodiments, the countertraction element 1000 may be delivered with the anchoring element 1002 or with the guide wire 902 through the right atrium and then positioned in the left atrium and pulled against the septum primum to provide countertraction. When the countertraction element is connected to an anchoring element, the anchoring element is delivered in the patent foramen ovale tunnel, and the countertraction element engages the septa past the tunnel in one of the atria. When the countertraction element is attached to guide wire 902, delivery can occur as described above. In such an embodiment, for example, the countertraction element may be delivered through the tunnel of the patent foramen ovale, or penetrated through the septa, such as described with the balloon embodiment above.

Once the countertraction element is in position adjacent one of the septa, a closure device, such as described with reference to Figure 6, may be delivered through the septum and through the cover, fixing the cover to the septum and thereby closing the shunt through the patent foramen ovale, as shown in Figures 26 and 27. For example, a coil as described above may be delivered through the cover to fix the cover to the septum primurn. When an anchoring element is used, the anchoring element 1002 may then be detached from the cover and removed with the delivery system. In some embodiments, the closure device permanently engages the anchoring element. Accordingly, the anchoring element may be releasably attached to the delivery device, such that the anchoring element is also used as a closure element and remains in the tunnel. As shown in Figures 26 and 27, in the final configuration, both the cover 1004 of the countertraction element 1000 and the closure device 300 are used to together hold and secure the septa of the patent foramen ovale.

In each of the embodiments described above, the countertraction element may comprise a bioabsorbable material. In some embodiments, the countertraction element is friable. In one embodiment, the countertraction element comprises PVA foam. In an embodiment wherein the countertraction element comprises a bioabsorbable material and is friable, the countertraction element can be freed from the implant if the countertraction element is anchored to the implant during the delivery process. Additionally, any material trapped by the implant during the delivery process will bio-resorb.

### Implant Positioning Systems and Methods

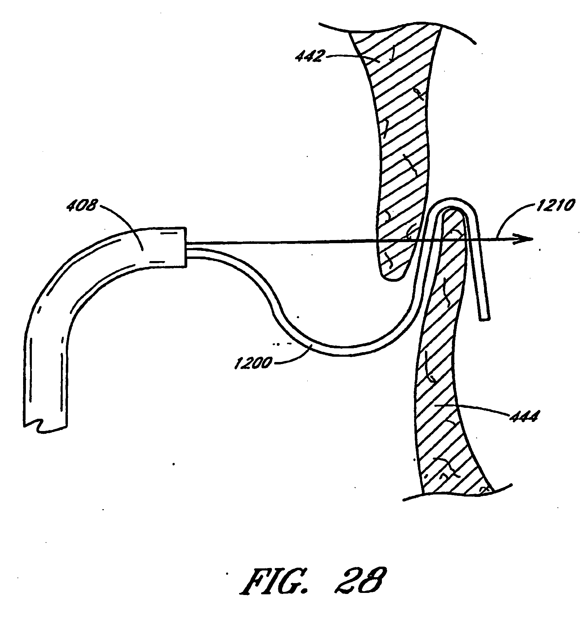

Figures 28-29B show systems and methods for positioning an implant at a patent foramen ovale using a flexible or pre-shaped guide wire 1200. A catheter 408 delivers the guide wire to the treatment site, preferably from the right atrium. As illustrated, the guide wire 1200 is passed through the tunnel of the patent foramen ovale, between and around the septum secundum 442 and septum primum 444. As shown in Figure 28, the guide wire may have a preshaped curve such as an S-shaped curve at its distal end for positioning around the tip of the septum secundum 442, through the tunnel of the patent foramen ovale, and around the septum primum 444. In one embodiment, the guide wire may be made of a superelastic material such as nickel titanium, and may be delivered in a straightened configuration through a catheter inserted through the tunnel of the patent foramen ovale. Once the catheter is delivered through the tunnel of the patent foramen ovale, the catheter can be retracted proximally relative to the guide wire or the guide wire can be advanced distally out of the catheter, and the guide wire can self-expand to its S-shaped configuration around the septum primum and septum secundum. Optionally, countertraction elements, as described above, can be attached to the distal end of the guide wire in order to create an increased surface area capable of providing countertraction against the septum primum. As illustrated, the guide wire 1200 itself acts as the countertraction element. In some embodiments, the guide wire structure has a ribbon-like cross-section. Other cross-sections which provide adequate maneuverability at the treatment site may be used.

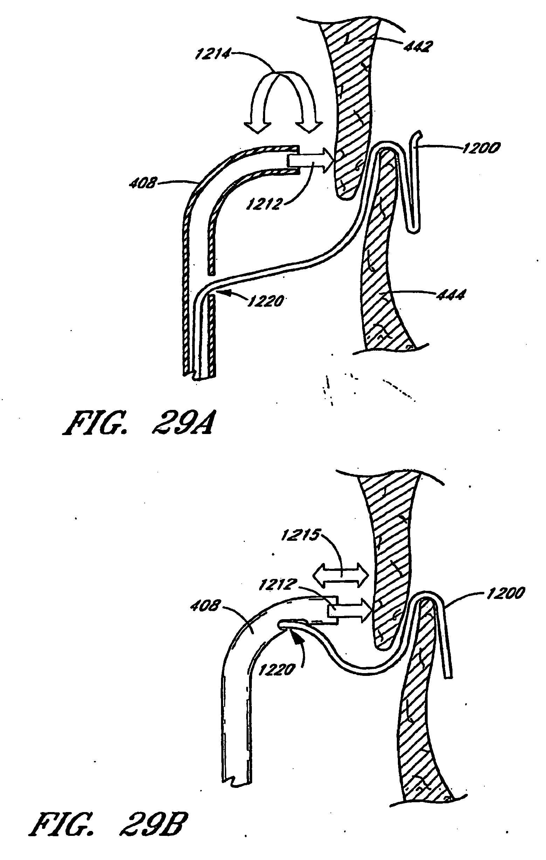

As shown in Figure 28, the guide wire 1200 is delivered through the same catheter 408 that is used to deliver an implant, such as the closure devices described above. The guide wire may be provided through the same lumen as the closure device, or a separate lumen. The trajectory of the implant is generally in the direction of arrow 1210. Figure 29A and 29B show other embodiments of a pre-shaped guide wire 1200 advanced through a side lumen 1220 in the catheter 408, just proximal to the distal end of catheter 408. The implant is directed through the septa of the patent foramen ovale following the trajectory 1212, which generally coincides with the longitudinal axis of the catheter 408. After the guide wire has been positioned, the catheter 408 can be rotated or repositioned, while the guide wire remains engaged within the patent foramen ovale, to offset the position of the countertraction element and the closure device. For example, the catheter 408 can be rotated or moved relative to the guide wire 1200 to deliver a closure device at locations on either side of the guide wire 1200. For example, Figure 29A illustrates the catheter 408 being repositioned according to arrows 1214 to deliver a closure device adjacent the guide wire and through the septum secundum and septum primum. As shown in Figure 29B, the catheter 408 can be advanced and retracted according to arrow 1215 to further reposition the catheter 408 in order to properly locate the position for delivering the closure device.

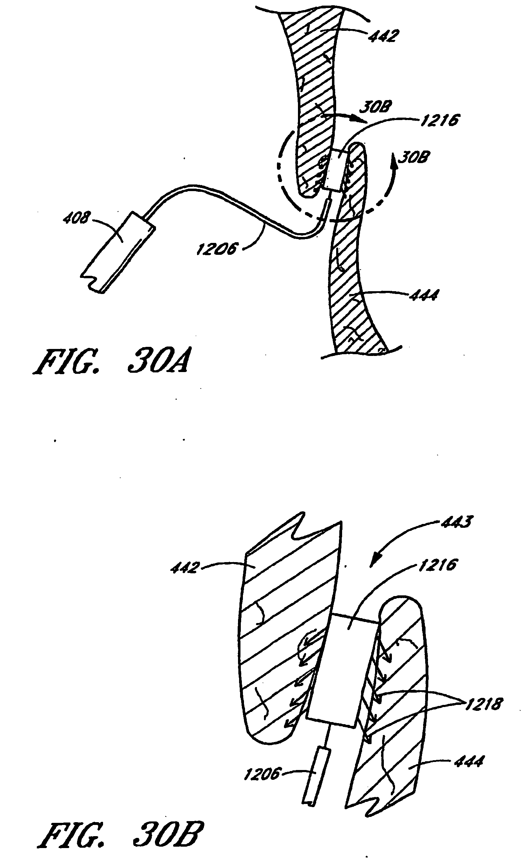

Figures 30A and 30B illustrate another embodiment of the invention wherein an implant 1216 is delivered into the tunnel between the septum primum and septum secundum of the patent foramen ovale. In some embodiments, the implant is removable. As illustrated, the implant may be carried on a guide wire 1206, other suitable delivery device, and may include barbs, anchors or other tissue retention structures 1218 for holding the septum primum and septum secundum together. In some embodiments, the barbs, anchors or other tissue retention structures 1218 may be retractable. In one embodiment wherein the tissue retention structures are retractable, the tubular implant comprises a plurality of radial holes arranged around its circumference at several points. An elongate core is slideably received within the tubular implant. The elongate core may comprise a plurality of protrusions extending radially away from the elongate core. The protrusions may be biased to expand radially. As the core slides distally through the tubular implant, the protrusions expand when exposed at the holes. The protrusions may be retracted by pushing or pulling the core relative to the tubular implant.

The implant 1216 may be releasably attached to the guide wire 1206, such as by using threading, snap fitting or other suitable mechanisms. Once the implant 1216 is in place holding the septum primum and septum secundum relative to one another, a closure device may be delivered to the patent foramen ovale, such as described in the embodiments above. The implant 1216 may be left in the patent foramen ovale after delivery of the closure device. It will be appreciated that the implant 1216 may also be designed to be retrievable by the guide wire 1206 or other method, and may also be designed to be temporarily held in place in the patent foramen ovale tunnel during delivery of the closure device.

While particular forms of the invention have been described, it will be apparent that various modifications can be made without departing from the scope of the invention. Accordingly, it is not intended that the invention be limited, except as by the appended claims.

## Claims

1. An assembly (400) for delivering a closure device (210. 300, 310, 1108) to a patent foramen ovale having a septum primum and a septum secundum, comprising:
a closure device (210, 300, 310, 1108) suitable for deployment at the patent foramen ovale to hold the septum primum and septum secundum together;
a delivery catheter (408) sized to receive the closure device and suitable for advancing the closure device to the patent foramen ovale from one side of the patent foramen ovale; and **characterized in that** the assembly further comprises
a countertraction element (900, 1100) suitable for positioning on the opposite side of the patent foramen ovale the countertraction element suitable for holding the position of at least one of the septa while the closure device is advanced into position.

2. The assembly of Claim 1 wherein the delivery catheter (408) is sized to receive both the closure device and the countertraction element and is suitable for advancing the countertraction element to the opposite side of the patent foramen ovale.

3. The assembly of any one of the preceding claims, wherein a distal end of the countertraction element (900, 1100) is sufficiently sharp to penetrate the septa.

4. The assembly of any of the preceding claims, wherein the countertraction element (90, 1100) is attached to a guide wire (902).

5. The assembly of Claim 4 further comprising a hollow tissue piercing structure suitable for piercing the septa to allow delivery of the guide wire (902) and countertraction element (900, 1100).

6. The assembly of any one of the preceding claims, wherein the countertraction element (900, 1100) is expandable from a retracted state to an expanded state.

7. The assembly of Claim 4 or 5, wherein the countertraction element (900, 1100) comprises an expanded, substantially planar state, wherein the plane of the countertraction element is substantially parallel to a first axis (B) extending through the countertraction element, the first axis (B) being perpendicular to a longitudinal axis (A) of the guide wire.

8. The assembly of anyone of the preceding claims, wherein the countertraction element (900,1100) comprises an inflatable balloon.

9. The assembly of any one of the preceding claims, wherein the countertraction element (900, 1100) comprises an expandable wire with one or more loops (906, 908, 910).

10. The assembly of any one of Claims 1-5, wherein the countertraction element comprises a snare-like element (956).

11. The assembly of Claim 1, further comprising a separate catheter suitable for delivering the countertraction element to the opposite side of the patent foramen ovale.

## Patentansprüche

1. Aufbau (400) für das Zuführen einer Verschlußvorrichtung (210, 300, 310, 1108) zu einem offenen Foramen ovale mit einem Septum primum und einem Septum secundum, der aufweist:
eine Verschlußeinrichtung (210, 300, 310, 1108), die für den Einsatz am offenen Foramen ovale geeignet ist, um das Septum primum und das Septum secundum zusammenzuhalten,
einen Zuführungskatether (408), der in der Größe dimensioniert ist, um die Verschlußeinrichtung aufzunehmen und geeignet ist für das Vorrücken der Verschlußeinrichtung zu dem offenen Foramen ovale von einer Seite des offenen Foramen ovale und **dadurch gekennzeichnet, dass** der Aufbau weiterhin aufweist:
ein Gegenzugelement (900, 1100), das geeignet ist für das Positionieren auf der gegenüberliegenden Seite des offenen Foramen ovale, wobei das Gegenzugelement geeignet ist für das Halten der Position von zumindest einer der Septa, während die Verschlußvorrichtung in die Position vorgerückt wird.

2. Aufbau nach Anspruch 1, bei dem der Zuführungskatether (408) in der Größe dimensioniert ist, um sowohl die Verschlußeinrichtung als auch das Gegenzugelement aufzunehmen, und geeignet ist für das Vorbewegen des Gegenzugelementes zur gegenüberliegenden Seite des offenen Foramen ovale.

3. Aufbau nach einem der vorherigen Ansprüche, bei dem ein distales Ende des Gegenzugelementes (900, 1100) ausreichend spitz ist, um die Septa zu durchdringen.

4. Aufbau nach einem der vorherigen Ansprüche, bei dem das Gegenzugelement (900, 1100) an einem Führungsdraht (902) befestigt ist.

5. Aufbau nach Anspruch 4, der weiterhin aufweist eine hohle gewebedurchstechende Struktur, die geeignet ist für das Durchdringen der Septa, um die Zuführung des Führungsdrahtes (902) und des Gegenzugelementes (900, 1100) zu ermöglichen.

6. Aufbau nach einem der vorherigen Ansprüche, bei dem das Gegenzugelement (900, 1100) erweiterbar ist von einem zurückgezogenen Zustand in einen erweiterten Zustand.

7. Aufbau nach Anspruch 4 oder 5, bei dem das Gegenzugelement (900, 1100) einen erweiterten, im wesentlichen ebenen Zustand, in dem die Ebene des Gegenzugelementes im wesentlichen parallel zu einer ersten Achse (B), die sich durch das Gegenzugelement erstreckt, aufweist, wobei die erste Achse (B) senkrecht zu einer Längsachse (A) des Führungsdrahtes ist.

8. Aufbau nach einem der vorherigen Ansprüche, wobei das Gegenzugelement (900, 1100) einen aufblasbaren Ballon aufweist.

9. Aufbau nach einem der vorherigen Ansprüche, bei dem das Gegenzugelement (900, 1100) einen verlängerbaren Draht mit ein oder mehreren Schleifen (906, 908, 910) aufweist.

10. Aufbau nach einem der Ansprüche 1 bis 5, bei dem das Gegenzugelemente ein schlingenartiges Element (956) aufweist.

11. Aufbau nach Anspruch 1, der weiterhin aufweist einen separaten Katether, der für das Zuführen des Gegenzugelementes zur gegenüberliegenden Seite des offenen Foramen ovale aufweist.

## Revendications

1. Ensemble (400) pour délivrer un dispositif de fermeture (210, 300, 310, 1108) à un foramen ovale perméable comportant un septum primum et un septum secundum, comprenant :
un dispositif de fermeture (210, 300, 310, 1108) approprié pour un déploiement au niveau du foramen ovale perméable pour maintenir le septum primum et le septum secundum ensemble,
un cathéter de distribution (408) dimensionné pour recevoir le dispositif de fermeture et approprié pour avancer le dispositif de fermeture vers le foramen ovale perméable à partir d'un côté du foramen ovale perméable ; et **caractérisé en ce que** l'ensemble comprend en outre :
un élément de contretraction (900, 1100) approprié pour un positionnement du côté opposé du foramen ovale perméable, l'élément de contretraction étant approprié pour maintenir la position d'au moins l'un des septums alors que le dispositif de fermeture est avancé en position.

2. Ensemble selon la revendication 1, dans lequel le cathéter de distribution (408) est dimensionné pour recevoir à la fois le dispositif de fermeture et l'élément de contretraction et est approprié pour avancer l'élément de contretraction vers le côté opposé du foramen ovale perméable.

3. Ensemble selon l'une quelconque des revendications précédentes, dans lequel une extrémité distale de l'élément de contretraction (900, 1100) est suffisamment pointue pour pénétrer dans les septums.

4. Ensemble selon l'une quelconque des revendications précédentes, dans lequel l'élément de contretraction (900, 1100) est fixé à un fil de guidage (902).

5. Ensemble selon la revendication 4, comprenant en outre une structure de percement de tissu creuse appropriée pour percer les septums pour permettre la distribution du fil de guidage (902) et de l'élément de contretraction (900, 1100).

6. Ensemble selon l'une quelconque des revendications précédentes, dans lequel l'élément de contretraction (900, 1100) est extensible d'un état rétracté vers un état étendu.

7. Ensemble selon la revendication 4 ou 5, dans lequel l'élément de contretraction (900, 1100) comprend un état sensiblement planaire, étendu, dans lequel le plan de l'élément de contretraction est sensiblement parallèle à un premier axe (B) s'étendant à travers l'élément de contretraction, le premier axe (B) étant perpendiculaire à un axe longitudinal (A) du fil de guidage.

8. Ensemble selon l'une quelconque des revendications précédentes, dans lequel l'élément de contretraction (900, 1100) comprend un ballonnet gonflable.

9. Ensemble selon l'une quelconque des revendications précédentes, dans lequel l'élément de contretraction (900, 1100) comprend un fil extensible avec une ou plusieurs boucles (906, 908, 910).

10. Ensemble selon l'une quelconque des revendications 1 à 5, dans lequel l'élément de contretraction comprend un élément de type anse (956).

11. Ensemble selon la revendication 1, comprenant en outre un cathéter séparé approprié pour délivrer l'élément de contretraction vers le côté opposé du foramen ovale perméable.
